# EUROPÄISCHE PATENTANMELDUNG

(11) **EP 3 922 916 A1**
(43) Veröffentlichungstag der Anmeldung: **15.12.2021**
(21) Anmeldenummer: 21178452.5
(22) Anmeldetag: 09.06.2021
(51) Int. Cl.: F24F 8/22, A61L 2/10, A61L 9/20

(54) **LUFTREINIGUNGSEINRICHTUNG**

(30) Priorität: 10.06.2020 DE 202020103352 U
(71) Anmelder: BÄ*RO GmbH & Co. KG, 42799 Leichlingen (DE)
(72) Erfinder: Miesner, Christian, 47918 Tönisvorst (DE)
(74) Vertreter: Paul & Albrecht Patentanwälte PartG mbB

(57) **Zusammenfassung**

Die vorliegende Erfindung betrifft eine Luftreinigungseinrichtung (1) für die Luftentkeimung mit einem Luftbehandlungsraum (2) und wenigstens einer Beleuchtungseinheit (3), die derart ausgebildet und angeordnet ist, dass mittels dieser UV-Strahlung in den Luftbehandlungsraum (2) gestrahlt werden kann, um zu reinigende Luft, die im Betrieb den Luftbehandlungsraum (2) durchströmt, mit dieser zu behandeln und so darin enthaltene Mikroorganismen, insbesondere Bakterien und/oder Viren und/oder Pilze, abzutöten oder zu inaktivieren, wobei der Luftbehandlungsraum (2) zumindest abschnittsweise zumindest im Wesentlichen kugelförmig ausgebildet ist und die den Luftbehandlungsraum (2) begrenzende Wandung (5) innenseitig zumindest abschnittsweise mit einer Reflexionsbeschichtung (9), die dazu ausgebildet ist, von der wenigstens einen Beleuchtungseinheit (3) emittierte UV-Strahlung bevorzugt diffus zu reflektieren, versehen ist.

## Beschreibung

Die vorliegende Erfindung betrifft eine Luftreinigungseinrichtung für die Luftentkeimung mit einem Luftbehandlungsraum und wenigstens einer Beleuchtungseinheit, die derart ausgebildet und angeordnet ist, dass mittels dieser UV-Strahlung in den Luftbehandlungsraum gestrahlt werden kann, um zu reinigende Luft, die im Betrieb den Luftbehandlungsraum durchströmt, mit dieser zu behandeln und so darin enthaltene Mikroorganismen, insbesondere Viren zu inaktivieren und/oder Bakterien und/oder Pilze, abzutöten oder zu inaktivieren. Darüber hinaus betrifft die Erfindung eine raumlufttechnische Anlage und eine Lüftungs- oder Klimaanlage mit einer solchen Einrichtung, ein Fahrzeug und eine Verwendung.

Zur Entkeimung von Luft, beispielsweise in gewerblich und industriell genutzten Räumen sowie in Krankenhäusern, werden UV-Strahler als Beleuchtungseinheiten eingesetzt. Ihre keimtötende UV-Strahlung wird verwendet, um Bakterien, Pilze, Viren oder andere Mikroorganismen abzutöten bzw. zu inaktivieren. Die UV-Strahler sind dabei in Luftreinigungseinrichtungen der eingangs genannten Art enthalten, die einen Luftbehandlungsraum aufweisen, durch den im Betrieb die zu reinigende Luft geführt wird und in den die UV-Strahler einstrahlen. Der Luftbehandlungsraum kann Teil eines beispielsweise zylinder- oder quaderförmigen Strömungskanals der Einrichtung sein oder einen solchen bilden. Luftreinigungsgeräte, die UV-Strahler, insbesondere UV-C-Strahler als Beleuchtungseinheiten zur Keimtötung verwenden, sind beispielsweise aus der DE 93 13 409 U, DE 26 18 127 A1, DE 32 08 519 A1 und der DE 199 06 113 A1 bekannt.

In den bekannten Einrichtungen kommen in der Regel Quecksilberniederdruckstrahler als Beleuchtungseinheiten zum Einsatz, um die UV-Strahlung bereitzustellen, mittels derer die vorbeiströmende Luft entkeimt wird. Photonen im Wellenlängenbereich von etwa 254 nm treffen auf in der Luft enthaltene Mikroorganismen, wie Bakterien und/oder Pilze und/oder Viren, und töten bzw. inaktivieren diese ab. Standard-UV-Strahler strahlen in allen Richtungen (4 Pi) ab. Photonen, die nicht mit Partikeln in der Luft kollidieren, werden an der bzw. den Innenwandungen des Luftbehandlungsraums teilweise reflektiert und im Wesentlichen absorbiert. An der Wandung absorbierte Photonen haben keine entkeimende Wirkung. Mit einem nicht unbeachtlichen Teil der aufgebrachten Lichtleistung geht daher keine entkeimende Wirkung einher bzw., um eine Entkeimung in zufriedenstellendem Maße zu erhalten, ist ein nicht unbeachtlicher Stromaufwand erforderlich.

Für einen entkeimenden Effekt könnten nach Auffassung der Anmelderin prinzipiell auch UV-Licht emittierende LEDs genutzt werden. UV-LEDs mit einer Wellenlänge von etwa 280 nm (+/- 5 nm) beispielsweise stehen zur Verfügung. Optimal für die Entkeimung ist eine Wellenlänge von 260 nm. UV-LEDs sind jedoch in der Regel ineffizienter als Niederdruckstrahler, was die optische zur elektrischen Leistung angeht.

Es ist daher eine Aufgabe der vorliegenden Erfindung, eine Luftreinigungseinrichtung für die Luftentkeimung der eingangs genannten Art anzugeben, die sie sich durch eine gute entkeimende Wirkung bei gleichzeitig moderatem Stromverbrauch auszeichnet.

Diese Aufgabe wird bei einer Luftreinigungseinrichtung für die Luftentkeimung der eingangs genannten Art dadurch gelöst, dass der Luftbehandlungsraum zumindest abschnittsweise zumindest im Wesentlichen kugelförmig ausgebildet ist und die den Luftbehandlungsraum begrenzende Wandung innenseitig zumindest abschnittsweise mit einer Reflexionsbeschichtung, die dazu ausgebildet ist, von der wenigstens einen Beleuchtungseinheit emittierte UV-Strahlung bevorzugt diffus zu reflektieren, versehen ist.

Der Grundgedanke der vorliegenden Erfindung besteht mit anderen Worten darin, die "Kontakt"- bzw. Wechselwirkungswahrscheinlichkeit zwischen von der wenigstens einen Beleuchtungseinheit emittierten Photonen und Partikeln der den Luftbehandlungsraum im Betrieb durchströmenden Luft gezielt durch eine geeignete Ausgestaltung des Luftbehandlungsraums zu erhöhen. Dieser ist erfindungsgemäß zumindest abschnittsweise zumindest im Wesentlichen kugelförmig ausgestaltet und an seiner Innenwandung mit einer Reflexionsbeschichtung versehen. Es liegt mit anderen Worten eine den Luftbehandlungsraum definierende Hohlkugel vor, die von innen mit einem (zumindest) im UV-Bereich bevorzugt diffus und/oder lambertsch reflektierenden Material ausgekleidet ist. Die Ausgestaltung ist derart getroffen, dass Strahlung, die von der oder wenigstens einer der Beleuchtungseinheiten emittiert wird, mehrfach an der den Luftbehandlungsraum begrenzenden, mit der Reflexionsbeschichtung versehenen Wandung reflektiert werden kann bzw. reflektiert wird. Sie kann so lange innerhalb der Kugel reflektiert werden (Multireflexion), bis es zu einem Kontakt mit einem Luftpartikel kommt. Wenn es sich bei dem Luftpartikel beispielsweise um ein Bakterium oder Virus handelt, wird dieses abgetötet bzw. inaktiviert und man spricht von Entkeimung. Im Ergebnis wird die Entkeimungswirkung erfindungsgemäß erhöht. Dies allein aufgrund der konstruktiven Ausgestaltung und ohne, dass damit ein erhöhter Stromverbrauch einhergeht.

Der Luftbehandlungsraum zeichnet sich bevorzugt über seine gesamte Ausdehnung durch eine kugelförmige Gestalt aus. Der Luftbehandlungsraum ist ferner insbesondere durch eine zumindest abschnittsweise zumindest im Wesentlichen hohlkugelförmige Wandung begrenzt, an deren Innenseite - zumindest abschnittsweise - die Reflexionsbeschichtung vorgesehen ist. Die den Luftbehandlungsraum begrenzende Wandung kann zumindest im Wesentlichen die Form einer vollen Hohlkugel haben. Die Wandung zeichnet sich mit anderen Worten bevorzugt über ihre gesamte Ausdehnung durch eine zumindest im Wesentlichen hohlkugelförmige Gestalt aus, diese ggf. allein mit Ausnahme von Bereichen, in denen eine oder mehrere Lufteinlass- und Luftauslassöffnung(en) bzw. eine oder mehrere Lichteinlassöffnung(en) vorliegen.

Die erfindungsgemäß vorgesehene Reflexionsbeschichtung ist zweckmäßiger Weise derart ausgebildet, dass sie die von der wenigstens einen Beleuchtungseinheit stammende Strahlung diffus reflektiert. Unter diffuser Reflexion ist insbesondere in hinlänglich vorbekannter Weise zu verstehen, dass das Licht maßgeblich nach dem lambertschen Gesetz reflektiert wird. Licht wird daher nicht (oder zumindest nicht nur) direkt reflektiert, sondern in verschiedene Richtungen zurückgeworfen und gestreut wird.

Das Prinzip der Multireflexion in einer Hohlkugel mit diffus reflektierender Innenbeschichtung ist aus dem Bereich optischen Messtechnik vorbekannt. Dort wird eine entsprechende Kugel auch als Ulbricht-Kugel bezeichnet (siehe zum Beispiel den Wikipedia-Artikel "Ulbricht-Kugel", aufrufbar unter https://de.wikipedia.org/wiki/Ulbricht-Kugel). Die Multireflexion wird hier zu einem anderen Zweck genutzt, insbesondere dazu, optische Strahlung (Lichtstrom) in allen Raumrichtungen mit einer Kurzzeitmessung zu erfassen. Eine Ulbricht-Kugel hat sich als besonders geeignet erwiesen, um auf die erfindungsgemäße Weise die Kontaktwahrscheinlichkeit zwischen Photonen und Luftpartikeln zu erhöhen und so eine gute Entkeimungswirkung zu gewährleisten.

Der Luftbehandlungsraum der erfindungsgemäßen Luftreinigungseinrichtung hat bevorzugt zumindest im Wesentlichen die Form einer vollen Kugel, mit anderen Worten Vollkugel. Der Luftbehandlungsraum kann eine Lufteinlassöffnung und eine der Lufteinlassöffnung bevorzugt gegenüberliegende Luftauslassöffnung aufweisen. Insbesondere im Bereich einer für den Lufteinlass in den Luftbehandlungsraum vorgesehenen Lufteinlass- und einer für den Luftauslass aus dem Luftbehandlungsraum vorgesehenen Luftauslassöffnung kann eine Abweichung von der Vollkugelform vorliegen. Es kann sein, dass eine Abweichung allein im Bereich einer Lufteinlass- und Luftauslassöffnung vorliegt.

Dann bildet der Luftbehandlungsraum mit andere Worten eine Ulbricht-Kugel. Dies ist ideal für eine Multireflexion und somit hohe Wechselwirkungswahrscheinlichkeit und gute Entkeimungsleistung.

Für eine besonders effiziente Entkeimung ist vorteilhafter Weise die den Luftbehandlungsraum definierenden bzw. begrenzende Wandung an ihrer kompletten Innenseite mit der Reflexionsbeschichtung versehen.

Der Durchmesser des Luftbehandlungsraumes kann beispielsweise im Bereich von wenigen mm bis hin zum mehreren Metern, abhängig vom Luftvolumen pro Stunde, liegen.

Der Durchmesser einer Lufteinlassöffnung, durch welche zu reinigende Luft in den Luftbehandlungsraum eintreten kann, ist im Verhältnis zum Durchmesser des Luftbehandlungsraumes bevorzugt kleiner, beträgt z.B. z.B. 1/10 bis 1/3 des Durchmessers des Luftbehandlungsraumes. Gleiches kann alternativ oder zusätzlich für eine Luftauslassöffnung gelten, durch welche gereinigte Luft den Luftbehandlungsraum verlassen kann.

Unter UV-Strahlung ist insbesondere solche aus dem Wellenlängenbereich von 100 nm bis 400 nm zu verstehen.

Eine weitere ganz besonders vorteilhafte Ausgestaltung der Erfindung zeichnet sich dadurch aus, dass die oder wenigstens eine der Beleuchtungseinheiten wenigstens eine LED umfasst, die bevorzugt ausgebildet ist, elektromagnetische Strahlung im UV-Bereich, insbesondere elektromagnetische Strahlung mit einer Wellenlänge im Wellenlängenbereich von 200 nm bis 400 nm, bevorzugt 200 nm bis 380 nm, besonders bevorzugt 230 nm bis 285 nm, zu emittieren. LEDs basierend auf dem Halbleitermaterial AIGaN beispielsweise entwickeln sich zunehmen positiv in Effizienz und Wirtschaftlichkeit und stellen ein Beispiel von LEDs dar, die im Rahmen der vorliegenden Erfindung zum Einsatz kommen können. Dennoch liegt die Effizienz der LED im Vergleich zu Niederdruckstrahler derzeit in der Regel noch bei ca. 10-15%. Die Erfindung hat sich als ganz besonders geeignet in Kombination mit LED-Leuchtmitteln als Quelle für die entkeimende UV-Strahlung erwiesen. Da durch den erfindungsgemäß vorgesehenen, kugelförmigen Luftbehandlungsraum mit der Reflexionsbeschichtung die Wechselwirkungswahrscheinlichkeit zwischen Photonen und Luftpartikeln im Vergleich zum Stand der Technik erhöht ist, kann ohne Weiteres auch eine etwaig geringere Effizienz von LEDs in Kauf genommen und erfindungsgemäß kompensiert werden. Im Ergebnis kann eine Luftreinigungseinrichtung erhalten werden, die sich durch eine gute Entkeimungswirkung bei gleichzeitig vertretbarem Energiebedarf und hoher Lebensdauer auszeichnet. Sie kann mit vergleichsweise geringem Aufwand gefertigt werden und ist sehr wartungsarm, so dass deutlich geringere Wartungskosten anfallen.

Ein weiterer Vorteil von LEDs besteht darin, dass von diesen Licht besonders gerichtet in den Halbraum abgestrahlt werden kann. Eine Beleuchtungseinheit mit einer oder mehreren LEDs kann daher auch gut im Bereich einer den Luftbehandlungsraum begrenzenden Wandung angeordnet werden und von der Seite her, ggf. auch von außen in diesen einstrahlen. Elektrische Anschlüsse und Kontakte müssen dann nicht im Luftstrom eingebaut werden und können unter niedrigeren Sicherheits- und Feuchtigkeitsschutzklassen kostengünstiger ausgeführt werden. Ebenso können Kühleinrichtungen außerhalb angebracht und definiert betrieben werden und unterliegen so nicht den stark volatilen Temperaturbedingungen des Luftstroms und werden so auch vor Verschmutzung der mitgeführten Partikel im Luftstrom geschützt.

Alternativ oder zusätzlich kann vorgesehen sein, dass die oder wenigstens eine der Beleuchtungseinheiten wenigstens einen Excimer-Strahler umfasst, der bevorzugt ausgebildet ist, elektromagnetische Strahlung im UV-Bereich, insbesondere elektromagnetische Strahlung mit einer Wellenlänge im Wellenlängenbereich von 170 nm bis 400 nm, bevorzugt 170 nm bis 380 nm, besonders bevorzugt 210 nm bis 285 nm, zu emittieren. Unter Excimer ist dabei in bekannter Weise die Kurzform für "excited dimer" bzw. übersetzt ins Deutsche "angeregter Dimer" zu verstehen. Der wenigstens eine Excimer-Strahler kann beispielswiese durch wenigstens eine Excimer-Lampe, insbesondere Excimer-Entladungslampe, gegeben sein. Welche Wellenlänge von einem Excimer-Strahler emittiert wird, hängt in hinlänglich vorbekannter Weise insbesondere von dem verwendeten Gas bzw. der verwendeten Gasmischung ab. Es gilt insbesondere, dass mit Xe₂ eine Wellenlänge von 172 nm, mit KrCI eine Wellenlänge von 222 nm, mit XeBr eine Wellenlänge von 282 nm und mit XeCI eine Wellenlänge von 308 nm erhalten werden kann. Je nachdem, welche Wellenlänge gewünscht ist, kann ein Excimer-Strahler mit entsprechendem Gas bzw. entsprechender Gasmischung vorgesehen sein. Als ganz besonders geeignet hat sich erwiesen, wenn die oder wenigstens eine der Beleuchtungseinheiten wenigstens einen Excimer-Strahler umfasst, der ausgebildet ist, elektromagnetische Strahlung mit einer Wellenlänge von 222 nm zu emittieren.

Es hat sich gezeigt, dass der erfindungsgemäß vorgesehene kugelförmige, reflektierende Luftbehandlungsraum insbesondere auch in Kombination mit einem oder mehreren Excimer-Strahlern zu hervorragenden Ergebnissen führt.

Alternativ oder zusätzlich kann vorgesehen sein, dass die oder wenigstens eine der Beleuchtungseinheiten wenigstens eine Quecksilberniederdrucklampe, insbesondere eine nicht ozonbildende Quecksilberniederdrucklampe umfasst oder durch eine solche gegeben ist. Die Erfindung kann mit anderen Worten auch mit den bisher für die Luftentkeimung verwendeten Lichtquellen zum Einsatz kommen, wobei auch in diesem Falle erheblich von der allein durch die konstruktive Ausgestaltung verbesserten Wechselwirkungswahrscheinlichkeit profitiert und eine gute Entkeimungswirkung bei reduziertem Energiebedarf erzielt werden kann.

Es sei angemerkt, dass in einer erfindungsgemäßen Einrichtung in der Regel entweder die LED-Technik oder einer oder mehrere Excimer-Strahler oder eine oder mehrere Quecksilberniederdrucklampen bzw. - strahler zum Einsatz kommen werden. Es ist aber natürlich auch nicht ausgeschlossen, dass zwei oder alle drei Beleuchtungstechniken in einer erfindungsgemäßen Einrichtung kombiniert werden.

Weiterhin sei angemerkt, dass emittierte elektromagnetische Strahlung ein Spektrum mit mehr als einer Wellenlänge umfassen kann. Wenn davon die Rede ist, dass elektromagnetische Strahlung mit einer gegebenen Wellenlänge emittiert wird, können entsprechend neben der einen genannten Wellenlänge weitere Wellenlängen im Spektrum vorhanden sein bzw. sind vorhanden. Wenn eine einzelne Wellenlänge genannt ist, kann es sich um die Zentralwellenlänge des Spektrums handeln.

Die Beleuchtungseinheit oder - im Falle mehrerer - die Beleuchtungseinheiten können ferner gegen Staub- und/oder Spritzwasser geschützt sein, was beispielsweise durch ein entsprechendes Gehäuse realisierbar ist.

Die Reflexionsbeschichtung ist in zweckmäßiger Ausgestaltung dazu ausgebildet, wenigstens 50 %, bevorzugt wenigstens 70 %, besonders bevorzugt wenigstens 90 %, ganz besonders bevorzugt wenigstens 95 % derjenigen Strahlung zu reflektieren, die von der oder wenigstens einer der Beleuchtungseinheiten emittiert wird. Eine Reflexionsgrad von mindestens 50 % hat sich als geeignet erwiesen. Es sei jedoch betont, dass es auch nicht ausgeschlossen ist, dass auf eine Beschichtung mit geringerem Reflexionsgrad zurückgegriffen wird.

Was die Reflexionsbeschichtung angeht, hat sich Polytetrafluorethylen (PTFE) als ganz besonders geeignet erwiesen. Als Beispiel kann hier das in der optischen Messtechnik etablierte Spektralon angeführt werden. In Weiterbildung kann entsprechend vorgesehen sein, dass die Reflexionsbeschichtung Polytetrafluorethylen umfasst oder aus Polytetrafluorethylen besteht. Es handelt sich bevorzugt um weißes und/oder optisches Polytetrafluorethylen, also um ein Material, das hoch diffus reflektierend über einen weiten spektralen Bereich angewendet werden kann. PTFE ist UV-beständig, mit Wasser reinigungsfähig und temperaturbeständig bis 260°C. Dadurch können Ablagerungen aus der Luft an den Oberflächen besonders einfach entfernt werden. PTFE zeichnet sich auch über gleichbleibend gute Reflexionseigenschaften über einen breiten Wellenlängenbereich aus. Im sichtbaren Spektralbereich kann man von ca. 99% ausgehen, während im UV-Bereich in der Regel immer noch 95% Reflektivität genutzt werden kann.

Ein weiteres geeignetes Material ist Bariumsulfat (BaSO₄). Entsprechend kann alternativ oder zusätzlich vorgesehen sein, dass die Reflexionsbeschichtung Bariumsulfat umfasst oder aus Bariumsulfat besteht.

Sowohl PTFE als auch Bariumsulfat kommen auch aus dem Stand der Technik vorbekannten Ulbricht-Kugeln als diffus reflektierende Materialien für die Innenbeschichtung zum Einsatz und haben sich dort bewährt. Sie sind auch für die vorliegende Erfindung sehr gut geeignet.

Alternativ oder zusätzlich kann vorgesehen sein, dass die Reflexionsbeschichtung mit Siliziumoxyd beschichtetes Aluminium, bevorzugt Reinstaluminium, umfasst oder daraus besteht. Mit Siliziumoxyd (Quarzglas/SiO2) beschichtetes Reinstaluminium findet in der Lichttechnik standardmäßig Verwendung. Als Beispiel sei hier das Miro12-Material von Alanod genannt. Hier ist der Reflexionsgrad mit ca. 70% deutlich geringer, aber die Herstellungskosten sind ebenso weitaus niedriger.

Es sei angemerkt, dass die Reflexionsbeschichtung natürlich auch eine Kombination von zwei oder mehr der vorgenannten und/oder anderen Materialien umfassen oder aus einer Kombination von zwei oder mehr der vorgenannten und/oder anderen Materialien bestehen kann. Selbstverständlich kann es auch sein, dass eine Reflexionsbeschichtung in Teilen, etwa abschnittsweise, ein Material oder eine Materialkombination umfasst oder aus einem Material oder einer Materialkombination besteht, und in Teilen, etwa abschnittsweise, ein anderes Material oder eine andere Materialkombination umfasst oder aus einem anderen Material oder einer anderen Materialkombination besteht.

Eine weitere bevorzugte Ausführungsform der erfindungsgemäßen Luftreinigungseinrichtung zeichnet sich ferner dadurch aus, dass innerhalb des Luftbehandlungsraumes gegenüber der Lufteinlassöffnung ein Einlass-Shutterelement angeordnet ist. Dann gilt bevorzugt, dass das Einlass-Shutterelement an seiner von der Lufteinlassöffnung abgewandten Seite mit einer Reflexionsbeschichtung, die dazu ausgebildet ist, von der wenigstens einen Beleuchtungseinheit emittierte UV-Strahlung bevorzugt diffus zu reflektieren, versehen ist.

In analoger Weise kann besonders bevorzugt gelten, dass innerhalb des Luftbehandlungsraumes gegenüber der Luftauslassöffnung ein Auslass-Shutterelement angeordnet ist. Ist ein Auslass-Shutterelement vorhanden, ist dieses in vorteilhafter Weiterbildung an seiner von der Luftauslassöffnung abgewandten Seite mit einer Reflexionsbeschichtung, die dazu ausgebildet ist, von der wenigstens einen Beleuchtungseinheit emittierte UV-Strahlung bevorzugt diffus zu reflektieren, versehen.

Optische Shutter können den Verlust optischer Strahlung aus dem Luftbehandlungsraum heraus und beispielweise in einen dem Luftbehandlungsraum vor- und/oder nachgeschalteten Strömungskanalabschnitt verhindern. Sind Shutter-Elemente, die auch als Blenden- oder Abblend-Elemente bezeichnet werden können, vorhanden, sind diese bevorzugt mit Abstand von der Lufteinlass- bzw. Luftauslassöffnung zur Kugelmitte hin angeordnet und verdecken bzw. übergreifen die jeweilige Öffnung - mit Abstand - bevorzugt vollständig. Während sich ausbreitende optische Strahlung auf die Shutter trifft und zurückreflektiert wird, kann Luft um die beabstandet zur jeweiligen Öffnung angeordneten Shutter-Elementen herum und somit an diesen vorbeiströmen. Der Luftein- und - austritt wird durch solche also nicht verhindert. Deren Ausdehnung, insbesondere Durchmesser, ist bevorzugt an die Ausdehnung, insbesondere den Durchmesser derjenigen Öffnung angepasst, die sie verdecken sollen. Deren Durchmesser kann beispielsweise dem Durchmesser der entsprechenden Öffnung entsprechen oder diesen leicht, beispielsweise um mindestens 10 %, bevorzugt mindesten 20 % übersteigen.

Das Einlass-Shutterelement ist bevorzugt derart ausgebildet und in dem Luftbehandlungsraum angeordnet, dass Luft insbesondere ausschließlich seitlich des Einlass-Shutterelementes an diesem vorbeiströmen kann. In analoger Weise ist das Auslass-Shutterelement bevorzugt derart ausgebildet und in dem Luftbehandlungsraum angeordnet, dass Luft insbesondere ausschließlich seitlich des Auslass-Shutterelementes an diesem vorbeiströmen kann. Bevorzugt sind das Einlass-Shutterelement und/oder das Auslass-Shutterelement derart ausgebildet und in dem Luftbehandlungsraum angeordnet, dass Luft zu mehreren, bevorzugt allen Seiten des bzw. jeweiligen Shutterelementes an diesem vorbeiströmen kann.

Zweckmäßiger Weise gilt, dass der Durchmesser des Einlass-Shutterelementes und/oder der Durchmesser des Auslass-Shutterelementes den Durchmesser unterschreitet, den der Luftbehandlungsraum in demjenigen Bereich aufweist, in welchem das bzw. das betreffende Shutterelement angeordnet ist.

Das Einlass-Shutterelement kann derart ausgebildet und in dem Luftbehandlungsraum angeordnet sein, dass um das Einlass-Shutterelement herum bzw. zwischen dem Einlass-Shutterelement und der den Luftbehandlungsraum definierenden Wandung ein ringförmiger Strömungskanal definiert wird. Dies kann in analoger Weise für das Auslass-Shutterelement gelten.

Ein weitere Ausführungsform zeichnet sich ferner dadurch aus, dass genau ein Einlass-Shutterelement vorgesehen ist, und/oder dass das Einlass-Shutterelement eben und/oder einteilig ausgebildet ist. In analoger Weise kann gelten, dass genau ein Auslass-Shutterelement vorgesehen ist, und/oder dass das Auslass-Shutterelement eben und/oder einteilig ausgebildet ist.

Weiter bevorzugt kann gelten, dass das Einlass-Shutterelement und/oder das Auslass-Shutterelement als Elemente ohne Öffnungen ausgebildet sind. Man könnte auch sagen, dass es sich dann um durchgehend oder geschlossen ausgebildete Elemente handelt. Das Einlass-Shutterelement und/oder das Auslass-Shutterelement sind insbesondere luftundurchlässig ausgebildet. Luft kann dann ausschließlich seitlich an dem bzw. dem jeweiligen Shutterelement vorbeiströmen, nicht jedoch durch dieses hindurchströmen.

Sind Shutter-Elemente vorhanden, kann in Weiterbildung vorgesehen sein, dass die oder wenigstens eine der Beleuchtungseinheiten zwischen dem Einlass-Shutterelement und dem Auslass-Shutterelement angeordnet ist, was sich als besonders geeignete Anordnung erwiesen hat.

Der Luftbehandlungsraum der erfindungsgemäßen Luftreinigungseinrichtung kann einen bevorzugt zentralen Abschnitt eines Strömungskanals der Einrichtung bilden. Der Strömungskanal kann dann einen dem Luftbehandlungsraum in Strömungsrichtung vorgeschalteten, bevorzugt zylinderförmigen Luftzuführabschnitt und einen dem Luftbehandlungsraum in Strömungsrichtung nachgeschalteten, bevorzugt zylinderförmigen Luftabführabschnitt umfassen.

Zweckmäßiger Weise ist der Durchmesser des Luftzuführabschnitts kleiner als der Durchmesser des Luftbehandlungsraumes. Dann wird sich durch das größere Raumvolumen innerhalb der Kugel der Luftstrom während der Behandlung verlangsamen. Durch diesen Effekt in Kombination mit Multireflexion der Photonen erhöht sich die Kontaktwahrscheinlichkeit zwischen Photon und Luftpartikel noch weiter. Dadurch steigert die Anordnung die Effizienz in Bezug auf elektrische Leistung zur Entkeimungswirkung weiter. Luftpartikel sind neben Verschmutzungen auch und vor allem Mikroorganismen wie Pilze, Viren und/oder Bakterien.

Alternativ oder zusätzlich kann vorgesehen sein, dass der Durchmesser des Luftabführabschnitts kleiner ist als der Durchmesser des Luftbehandlungsraumes.

Ist der Luftbehandlungsraum Teil eines Strömungskanals mit einem vorgeschalteten Luftzuführabschnitt und einem nachgeschalteten Luftzuführabschnitt gilt weiter bevorzugt, dass der Luftzuführabschnitt in die Lufteinlassöffnung des Luftbehandlungsraums mündet und der Luftabführabschnitt von der Luftauslassöffnung des Luftbehandlungsraums abgeht.

In weiterer zweckmäßiger Ausgestaltung ist ein Gebläse vorgesehen, mittels dem im Betrieb zu reinigende Luft durch den Luftbehandlungsraum bzw. durch einen den Luftbehandlungsraum umfassenden Strömungskanal der erfindungsgemäßen Einrichtung gefördert werden kann.

Die oder wenigstens eine der Beleuchtungseinheiten kann innerhalb des Luftbehandlungsraumes angeordnet sein, beispielsweise zumindest in etwa mittig in dem Luftbehandlungsraum positioniert sein. Sie kann sich auch nahe oder unmittelbar an der den Luftbehandlungsraum begrenzenden Wandung befinden und beispielsweise auch an dieser befestigt sein. Dann findet die Einstrahlung nicht von der Mitte, sondern von der Seite her statt.

Befindet sich die oder wenigstens eine der Beleuchtungseinheiten bevorzugt in dem Luftbehandlungsraum, ist sie insbesondere dazu ausgebildet, in alle Richtungen, mit anderen Worten in den Vollraum (4pi) zu emittieren.

Die oder wenigstens eine der Beleuchtungseinheiten kann auch außerhalb des Luftbehandlungsraumes angeordnet sein, beispielsweise direkt neben oder an der Außenwandung des Luftbehandlungsraumes. Befindet sich die oder wenigstens eine Beleuchtungseinheit außerhalb des Luftbehandlungsraumes, ist zweckmäßiger Weise wenigstens eine Lichteinlassöffnung in der den Luftbehandlungsraum begrenzenden Wandung vorgesehen, durch welche von der oder der wenigstens einen der außerhalb angeordneten Beleuchtungseinheiten emittierte Strahlung in den Luftbehandlungsraum eingestrahlt werden kann.

In Weiterbildung kann dann vorgesehen sein, dass wenigstens ein optisches Medium, insbesondere wenigstens ein Lichtleiter, bevorzugt Glasfaserkabel der Glasstab, vorgesehen ist. Über das wenigstens eine optische Medium kann Strahlung, die von der oder wenigstens einer der außerhalb des Luftbehandlungsraumes angeordneten Beleuchtungseinheiten emittiert wird, in den Luftbehandlungsraum geführt werden. Über das wenigstens eine optische Medium kann mit anderen Worten eine lichtleitende Kopplung zwischen der oder wenigstens einer außerhalb des Luftbehandlungsraumes angeordneten Beleuchtungseinheit und dem Luftbehandlungsraum erzielt werden. Dazu ist beispielsweise ein Ende des optischen Mediums, etwa Glasfaserkabels, mit der oder wenigstens einer Beleuchtungseinheit verbunden und das andere freie Ende mit dem Luftbehandlungsraum, insbesondere der diesen begrenzenden Wandung. An der Wandung kann außenseitig ein Anschluss für eine optisches Medium, etwa ein Glasfaserkabel-Anschluss vorgesehen sein. Aus dem optischen Medium an diesem Ende austretende Infrarotstrahlung kann dann in den Luftbehandlungsraum über eine in der Wandung vorgesehene Öffnung eintreten und in diesem vielfach reflektiert werden.

Ist eine Beleuchtungseinheit bzw. sind Beleuchtungseinheiten außerhalb des Luftbehandlungsraumes angeordnet, ist diese bzw. sind diese nicht dem Luftstrom durch den Luftbehandlungsraum ausgesetzt und ist besonders gut zugänglich und kann bei Bedarf sehr einfach gewartet bzw. ausgetauscht werden. Ein weiterer Vorteil der Anordnung außerhalb kann darin bestehen, dass die Beleuchtungseinheit den Strahlengang in dem Luftbehandlungsraum, konkret die Multireflexionen, nicht blockiert.

Es sei angemerkt, dass für den Fall, dass eine oder mehrere Komponenten innerhalb des Luftbehandlungsraumes angeordnet sind, diese bevorzugt ebenfalls - zumindest abschnittsweise - mit einer Reflexionsbeschichtung versehen sind, die dazu ausgebildet ist, von der wenigstens einen Beleuchtungseinheit emittierte UV-Strahlung bevorzugt diffus zu reflektieren, wodurch absorptionsbedingte Verluste verhindert oder zumindest reduziert werden können. Als Beispiele für etwaig innerhalb des Luftbehandlungsraumes angeordnete Komponenten seien neben einer oder mehreren Beleuchtungseinheiten bzw. Gehäuseteilen dieser eine oder mehrere elektrische Zuleitungen für diese, eine oder mehrere Halterungen für diese, Fassungen sowie gegebenenfalls vorhandene Shutterelemente genannt. Die Reflexionsbeschichtung gegebenenfalls im Luftbehandlungsraum vorhandener Komponenten kann dann zu derjenigen an der Wandung des Luftbehandlungsraumes identisch oder auch von dieser verschieden ausgebildet sein. So kann beispielsweise die Wandung mit PTFE und innenliegende Shutter, Halterungen, Fassungen und Gehäuseteile mit Bariumsulfat beschichtet sein.

Es kann auch wenigstens eine der wenigstens einen Beleuchtungseinheit zugeordnete Kühleinrichtung vorgesehen sein. Auch für diese gilt bevorzugt, dass sie außerhalb des Luftbehandlungsraumes angeordnet ist. Eine Kühleinrichtung kann in die bzw. die jeweilige Beleuchtungseinheit integriert oder separat zu dieser ausgebildet sein.

Weiterhin kann wenigstens eine der wenigstens einen Beleuchtungseinheit zugeordnete Anschlussleitung vorgesehen sein. Auch für diese kann bevorzugt gelten, dass sie außerhalb des Luftbehandlungsraumes angeordnet ist.

Insbesondere für den Fall, dass die oder - im Falle mehrerer - wenigstens eine Beleuchtungseinheit außerhalb des Luftbehandlungsraumes vorgesehen ist, gilt diese besonders bevorzugt auch für deren Anschlussleitungen und eine dieser ggf. zugeordnete Kühleinrichtung.

Die erfindungsgemäße Luftreinigungseinrichtung für die Luftentkeimung eignet sich für die verschiedensten Anwendungsgebiete. Sie kann überall dort genutzt werden, wo eine Entkeimung der Luft vorteilhaft oder erforderlich ist. Sie kann zum Beispiel Bestandteil einer raumlufttechnischen Anlage, etwa einer raumlufttechnischen Anlage im Sinne von VDI6022, Stand Januar 2018, sein. Sie kann einen Bestandteil einer Lüftungs- oder Klimaanlage bilden.

Gegenstand der Erfindung ist auch eine raumlufttechnische Anlage, die wenigstens eine erfindungsgemäße Luftreinigungseinrichtung umfasst.

Die Erfindung betrifft ferner eine Lüftungs- oder Klimaanlage, die wenigstens eine erfindungsgemäße Luftreinigungseinrichtung umfasst.

Die erfindungsgemäße Luftreinigungseinrichtung bzw. eine raumlufttechnische Anlage bzw. Klima- oder Lüftungsanlage mit wenigstens einer solchen eignet sich sowohl für den stationären Bereich, kann beispielsweise in einem Gebäude vorgesehen sein, als auch für den mobilen Bereich, etwa als Bestandteil eines Fahrzeugs.

Die Erfindung betrifft daher auch ein Fahrzeug, insbesondere einen PKW oder LKW oder Bus oder eine Flugzeug oder Schiff, umfassend wenigstens eine erfindungsgemäße Luftreinigungseinrichtung. Die Erfindung kann mit anderen Worten auch in Fahrzeugen zum Einsatz kommen, sowohl in Landals auch Luft- als auch Wasserfahrzeugen. Eine oder mehrere erfindungsgemäße Luftreinigungseinrichtungen können beispielsweise Bestandteile einer oder mehrerer Lüftungs- oder Klimaanalgen, in einem Land-, Luft- oder Wasserfahrzeug sein.

Schließlich betrifft die Erfindung die Verwendung einer Ulbricht-Kugel in einer Luftreinigungseinrichtung für die Luftentkeimung mittels UV-Strahlung.

Hinsichtlich der Ausgestaltungen der Erfindung wird auch auf die Unteransprüche sowie auf die nachfolgende Beschreibung mehrerer Ausführungsbeispiele unter Bezugnahme auf die beiliegende Zeichnung verwiesen.

In der Zeichnung zeigt:
- Figur 1: ein erstes Ausführungsbeispiel einer erfindungsgemäßen Luftreinigungseinrichtung in rein schematischer Teilschnittdarstel-lung, wobei die Beleuchtungseinheit etwa mittig innerhalb des Luftbehandlungsraumes angeordnet ist;
- Figur 2: ein zweites Ausführungsbeispiel einer erfindungsgemäßen Luftreinigungseinrichtung in rein schematischer Teilschnittdarstellung, wobei die Beleuchtungseinheit außerhalb des Luftbehandlungsraumes angeordnet ist;
- Figur 3: ein drittes Ausführungsbeispiel einer erfindungsgemäßen Luftreinigungseinrichtung in rein schematischer Teilschnittdarstellung, wobei die Beleuchtungseinheit außerhalb des Luftbehandlungsraumes angeordnet und über ein optisches Medium, konkret ein Glasfaserkabel mit dem Luftbehandlungsraum gekoppelt ist; und
- Figur 4: ein Ausführungsbeispiel eines erfindungsgemäßen Fahrzeugs in rein schematischer Teilschnittdarstellung.

In den Figuren sind gleiche Komponenten mit gleichen Bezugszeichen versehen.

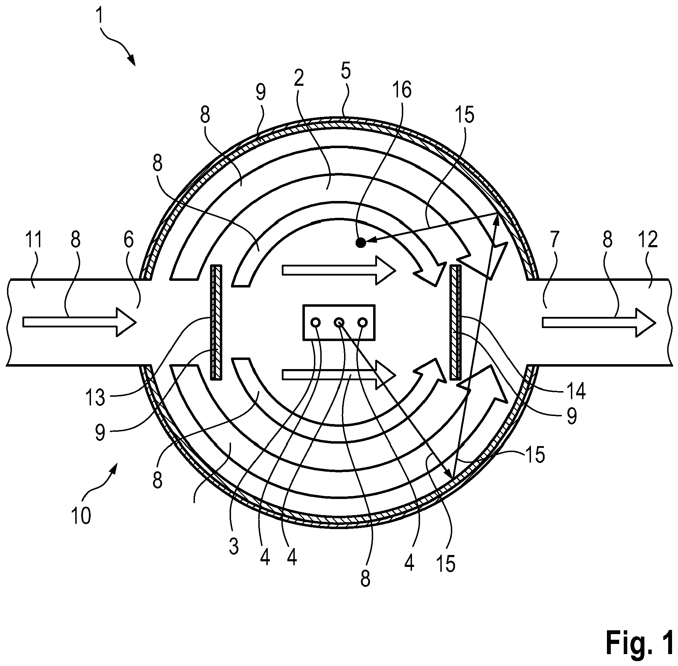

Die Figur 1 zeigt in rein schematischer Schnittdarstellung ein erstes Ausführungsbeispiel einer erfindungsgemäßen Luftreinigungseinrichtung 1 für die Luftentkeimung.

Die Luftreinigungseinrichtung 1 umfasst einen Luftbehandlungsraum 2 und eine Beleuchtungseinheit 3, die derart ausgebildet und angeordnet ist, dass mittels dieser UV-Strahlung in den Luftbehandlungsraum 2 gestrahlt werden kann, um zu reinigende Luft, die im Betrieb den Luftbehandlungsraum 2 durchströmt, mit dieser zu behandeln und so darin enthaltene Mikroorganismen, insbesondere Viren zu inaktivieren und/oder Bakterien und/oder Pilze, abzutöten. Wie man erkennt, ist die Beleuchtungseinheit 3 hier innerhalb des Luftbehandlungsraumes 2 angeordnet, befindet sich konkret in etwa mittig in diesem. Es ist eine Halterung vorgesehen, über welche die Beleuchtungseinheit 3 mittig in dem Luftreinigungsraum 2 gehalten wird.

Es sei angemerkt, dass alternativ dazu, dass die Beleuchtungseinheit 3 mittig in dem Luftbehandlungsraum 2 angeordnet ist, sich diese prinzipiell auch an anderer Stelle innerhalb dieses befinden kann.

Die Beleuchtungseinheit 3 ist vorliegend als LED-Beleuchtungseinheit 3 ausgebildet. Sie umfasst eine Mehrzahl von LEDs 4, die jeweils ausgebildet sind, UV-Licht, vorliegend Licht mit einer Wellenlänge von etwa 280 nm zu emittieren. Vorliegend handelt es sich um AIGaN-LEDs 4, wobei dies rein beispielhaft zu verstehen ist. Die Beleuchtungseinheit 3 ist ausgebildet, um in alle Richtungen (4 pi) abzustrahlen. Da die Beleuchtungseinheit LEDs umfasst, zeichnet sie sich durch eine hohe Lebensdauer und einen geringen Wartungsaufwand aus. Alternativ oder zusätzlich zu der LED-Technik können prinzipiell auch andere Beleuchtungsarten im Rahmen der Erfindung zum Einsatz kommen, etwa eine oder mehrere Quecksilberniederdrucklampen. Auch hat es sich als geeignet erwiesen, wenn die oder im Falle mehrerer wenigstens eine der Beleuchtungseinheiten 3 einen Excimer-Strahler umfasst. Als besonders geeignet haben sich beispielsweise Excimer-Strahler erwiesen, die dazu ausgebildet sind, UV-Licht mit einer Wellenlänge von 222 nm zu emittieren.

Die Beleuchtungseinheit 3 kann gegen Staub- und Spritzwasser geschützt sein, was vorliegend der Fall ist. Deren Gehäuse ist entsprechend ausgestaltet. Die Beleuchtungseinheit 3 umfasst eine Kühleinrichtung, mittels derer sie in hinlänglich vorbekannter Weise im Betrieb auf einer geeigneten Betriebstemperatur gehalten werden kann. Es ist ferner eine elektrische Zuleitung vorgesehen, über welche die Beleuchtungseinheit 3 mit einer Stromquelle verbindbar bzw. verbunden ist, so dass sie in ebenfalls hinlänglich vorbekannter Weise im Betrieb mit elektrischer Energie gespeist werden kann.

Der Luftbehandlungsraum 2 ist kugelförmig ausgebildet, hat konkret die Form einer Vollkugel. Der Luftbehandlungsraum 1 ist dabei durch eine im Wesentlichen hohlkugelförmige Wandung 5 begrenzt. Eine Abweichung von der vollen Hohlkugelform der Wandung 5 liegt, wie man erkennt, nur im Bereich einer Lufteinlassöffnung 6 und einer der Lichteinlassöffnung 6 gegenüberliegenden Luftauslassöffnung 7 vor, durch welche im Betrieb zu reinigende Luft in den Luftbehandlungsraum 2 gelangen bzw. gereinigte Luft diesen wieder verlassen kann. Die sich im Betrieb einstellende Luftströmung ist durch Pfeile 8 schematisch angedeutet.

Die den Luftbehandlungsraum 2 begrenzende Wandung 5 ist innenseitig mit einer Reflexionsbeschichtung 9 versehen, die dazu ausgebildet ist, von der Beleuchtungseinheit 3 im Betrieb emittierte UV-Strahlung diffus zu reflektieren. Die hohlkugelförmige Wandung 5 ist bei dem dargestellten Ausführungsbeispiel an ihrer kompletten Innenseite mit der Reflexionsbeschichtung 9 versehen. Die Reflexionsbeschichtung 9 ist weiß und besteht vorliegend aus Polytetrafluorethylen (PTFE), konkret optischem PTFE. PTFE kann mit Wasser gereinigt werden, so dass betriebsbedingte Verschmutzungen einfach entfernt werden können und bis 260°C temperaturbeständig. Die Reflexionsbeschichtung 9 ist vorliegend dazu ausgebildet wenigstens 95 %, insbesondere 97 % der von der Beleuchtungseinheit 3 emittierten UV-Strahlung zu reflektieren. Es sei betont, dass ein derart hoher Reflexionsgrad vorteilhaft ist, aber auch niedrigere Reflexionsgrade in Frage kommen.

Ebenfalls mit einer Reflexionsbeschichtung 9 versehen sind abschnittsweise das Gehäuse der Beleuchtungseinheit 3, die der Beleuchtungseinheit 3 zugeordnete Kühleinrichtung, die elektrische Zuleitung zur elektrischen Versorgung der Beleuchtungseinheit 3 und die Halterung, über welche diese mittig in dem Luftbehandlungsraum 2 gehalten wird. Bei dem dargestellten Ausführungsbeispiel ist die Reflexionsbeschichtung 9 auf diesen Komponenten identisch zu derjenigen an der Innenwandung ausgebildet, wobei dies rein beispielhaft zu verstehen ist. Bevorzugt gilt, dass alle in dem Luftreinigungsraum 2 angeordnete Komponenten mit einer Reflexionsbeschichtung 9 versehen sind.

Es sei angemerkt, dass als alternative Position der Beleuchtungseinheit 3 mittig innerhalb des Luftreinigungsraumes 2 die Beleuchtungseinheit 3 beispielsweise auch an der Wandung 5 anliegen und direkt an dieser befestigt sein könnte.

Die hohlkugelförmige Wandung 5 mit der Reflexionsbeschichtung 9 bildet eine Ulbricht-Kugel, wie sie aus dem Bereich der optischen Messtechnik prinzipiell vorbekannt ist. Mit dieser wird in der Luftreinigungseinrichtung leine hohe "Kontakt"- bzw. Wechselwirkungswahrscheinlichkeit zwischen von der Beleuchtungseinheit 3 im Betrieb emittierten Photonen und Partikeln der den Luftbehandlungsraum 2 im Betrieb durchströmenden Luft erzielt, wie weiter unten noch näher erläutert wird.

Der Luftbehandlungsraum 2 bildet einen zentralen Abschnitt eines Strömungskanals 10 der Luftreinigungseinrichtung 1. Dem zentralen Luftbehandlungsraum 2 in Strömungsrichtung vorgeschaltet ist ein zylinderförmiger Luftzuführabschnitt 11 des Strömungskanals 10 und dem Luftbehandlungsraum 2 in Strömungsrichtung nachgeschalteten ist ein ebenfalls zylinderförmiger Luftabführabschnitt 12 des Strömungskanals 10. Der Luftzuführabschnitt 11, über den im Betrieb dem Luftbehandlungsraum 2 zu reinigende Luft zugeführt wird, mündet in die Lufteinlassöffnung 6 des Luftbehandlungsraums 2 und der Luftabführabschnitt 12, über den im Betrieb aus dem Luftbehandlungsraum 2 austretende gereinigte Luft wieder abgeführt werden kann, geht von der Luftauslassöffnung 7 des Luftbehandlungsraums 2 ab. Wie man erkennt, ist der Durchmesser des Luftzuführabschnitts 11 kleiner als der Durchmesser des Luftbehandlungsraumes 2 und dies gilt ebenfalls für den Durchmesser des Luftabführabschnitts 12. Der Durchmesser des Luftbehandlungsraums 2 ist etwa drei Mal so groß wie der Durchmesser des Luftzuführabschnitts 11 und auch des Durchmessers des Luftabführabschnitts 12, der hier mit demjenigen des Luftzuführabschnitts 11 übereinstimmt.

Innerhalb des Luftbehandlungsraumes 2 gegenüber der Lufteinlassöffnung 6 ist ein Einlass-Shutterelement 13 angeordnet. Das Einlass-Shutterelement 13 ist an seiner von der Lufteinlassöffnung 6 abgewandten, in der Figur nach rechts weisenden Seite mit einer Reflexionsbeschichtung 9 versehen, die dazu ausgebildet ist, von der Beleuchtungseinheit 3 emittierte UV-Strahlung diffus zu reflektieren. In analoger Weise ist innerhalb des Luftbehandlungsraumes 2 gegenüber der Luftauslassöffnung 7 ein Auslass-Shutterelement 14 angeordnet, welches an seiner von der Luftauslassöffnung 7 abgewandten, in der Figur nach links weisenden Seite mit einer Reflexionsbeschichtung 9 versehen ist, die dazu ausgebildet ist, von der Beleuchtungseinheit 3 emittierte UV-Strahlung diffus zu reflektieren. Die Reflexionsbeschichtung 9 des Einlass-Shutterelementes 13 und des Auslass-Shutterelementes 14 ist bei dem gezeigten Beispiel zu der Reflexionsbeschichtung 9 an der Innenseite der den Luftbehandlungsraum 2 definierenden Wandung 5 identisch. Sie besteht somit ebenfalls aus PTFE und hat ein Reflexionsvermögen von 97% für die von der Beleuchtungseinheit 3, konkret deren LEDs 4 emittierte UV-Strahlung.

Wie man in der Figur sieht, sind genau ein Einlass-Shutterelement 13 und genau ein Auslass-Shutterelement 14 vorgesehen und das Einlass-Shutterelement 13 liegt näher an der Einlassöffnung 6 und das Auslass-Shutterelement 14 näher an der Auslassöffnung 7. Das Einlass-Shutterelement liegt bei dem dargestellten Beispiel näher an der Einlassöffnung 6 als am Mittelpunkt des kugelförmigen Luftbehandlungsraumes 2. In analoger Weise liegt das Auslass-Shutterelement 14 näher an der Auslassöffnung 7 als an dem Kugelmittelpunkt.

Das Einlass-Shutterelement 13 und das Auslass-Shutterelement 14 sind derart ausgebildet und in dem Luftbehandlungsraum 2 angeordnet, dass Luft seitlich des jeweiligen Shutterelementes 13, 14 an diesem vorbeiströmen kann. Wie man erkennt, unterschreitet der Durchmesser des Einlass-Shutterelementes 13 den Durchmesser, den der Luftbehandlungsraum 2 in demjenigen Bereich aufweist, in welchem dieses Shutterelement 13 angeordnet ist. Gleiches gilt für das Auslass-Shutterelement 14. Die Shutterelemente 13, 14 erstrecken sich nicht bis zur Wandung 5.

Analog zu der Halterung für die Beleuchtungseinheit 3 können auch Halterungen für die Shutterelemente 13, 14 vorgesehen sein.

Das Einlass-Shutterelement 13 ist insbesondere derart ausgebildet und in dem Luftbehandlungsraum 2 angeordnet, dass um das Einlass-Shutterelement 13 herum bzw. zwischen dem Einlass-Shutterelement 13 und der den Luftbehandlungsraum 2 definierenden Wandung 5 ein ringförmiger Strömungskanal definiert wird. Für das Auslass-Shutterelement 14 gilt dies bevorzugt gleichermaßen.

Das Einlass-Shutterelement 13 und das Auslass-Shutterelement 14 sind jeweils eben, einteilig und als Elemente, die keine Öffnungen aufweisen, ausgebildet. Es handelt sich um durchgehend bzw. geschlossen ausgebildete Elemente 13, 14. Die Shutterelemente sind luftundurchlässig. Luft kann ausschließlich seitlich an dem jeweiligen Shutterelement 13, 14 vorbeiströmen, nicht jedoch durch dieses hindurchtreten.

Die optischen Shutter 13, 14 verhindern den Verlust optischer Strahlung aus dem Luftbehandlungsraum 2 heraus in die vor- und nachgeschalteten Strömungskanalabschnitte 11 und 12 und tragen somit zu einer guten Effizienz bei.

Die Shutter-Elemente 13, 14 sind mit Abstand von der Lufteinlass- bzw. Luftauslassöffnung 6, 7 zur Kugelmitte hin angeordnet und verdecken bzw. übergreifen die jeweilige Öffnung 6,7 - mit Abstand - vollständig. Der Abstand kann beispielsweise 50% des Durchmessers des Lufteinlassöffnung 6 betragen. Der Durchmesser der Shutter-Elemente 13, 14 ist etwas größer als der Durchmesser der Lufteinlass- und Luftauslassöffnung 6, 7, beispielsweise um 20 %. Wie man erkennt, fluchten die Shutterelemente 13, 14 bei den gezeigten Beispielen mit der jeweiligen Öffnung 6, 7, der sie zugeordnet sind, und miteinander.

Während sich ausbreitende optische Strahlung im Betrieb auf die Shutterelemente 13, 14 trifft und aufgrund der Beschichtung 9 zurückreflektiert wird, kann Luft seitlich an den beabstandet zur jeweiligen Öffnung 6, 7 angeordneten Elementen 13, 14 vorbeiströmen, wie mit den Pfeilen 8 für den Luftstrom schematisch angedeutet. Der Luftein- und - austritt in den bzw. aus dem Luftbehandlungsraum 2 wird durch die Shutter 13, 14 also nicht verhindert, sondern nur der Strahlungsverlust.

Aus der Kugelform des Luftbehandlungsraumes 2 zusammen mit der Ausgestaltung und Anordnung der Shutterelemente 13, 14 ergibt sich die erkennbare, besonders vorteilhafte Strömungssituation (vgl. die Pfeile 8 in den Figuren). Die Luft wird seitlich der Shutterelemente 13, 14, deren Durchmesser den Durchmesser des kugelförmigen Luftbehandlungsraumes 2 an entsprechender Stelle jeweils unterschreitet, und entlang der gekrümmten, kugelförmigen Wandung 5, die den Luftbehandlungsraum 2 definiert, geführt. Die Luft gelangt auf optimale Weise von der Lufteinlassöffnung 6 zu der Luftauslassöffnung 7.

Die Beleuchtungseinheit 3 ist, wie man erkennt, mittig zwischen dem Einlass-Shutterelement 13 und dem Auslass-Shutterelement 14 angeordnet.

Im Betrieb strömt Luft durch den Strömungskanal 10 und somit nacheinander durch den Luftzuführabschnitt 11, den Luftbehandlungsraum 2 und den Luftabführabschnitt 12. Die Luft wird dabei mittels eines Gebläses der Luftreinigungseinrichtung 1 durch den Strömungskanal 10 gefördert.

Gleichzeitig wird von der in dem Luftbehandlungsraum 2 angeordneten, im Betrieb aktivierten Beleuchtungseinheit 3, konkret deren LEDs 4 UV-Strahlung emittiert. Die Strahlung wird mehrfach an der den Luftbehandlungsraum 2 begrenzenden, mit den Reflexionsbeschichtung 9 versehenen Wandung 5 reflektiert. Ein zugehöriger Strahlengang ist mit Pfeilen 15 rein schematisch in der Figur 1 angedeutet. Die Strahlung kann so lange innerhalb der Kugel reflektiert werden (Multireflexion), bis es zu einem Kontakt mit einem Luftpartikel 16 kommt. In der Figur ist rein beispielhaft nur eines der vielen im Betrieb im Luftbehandlungsraum 2 vorhandenen Luftpartikel 16 dargestellt. Wenn es sich bei dem Luftpartikel 16 beispielsweise um ein Bakterium oder Pilz oder Virus handelt, wird dieses abgetötet bzw. inaktiviert und man spricht von Entkeimung.

Da der Durchmesser des Strömungskanals 10 im Bereich des Luftzuführabschnittes 11 deutlich kleiner ist als der Durchmesser des kugelförmigen Luftbehandlungsraumes 2 wird sich aufgrund des größere Raumvolumens innerhalb der Kugel dabei der Luftstrom während der Behandlung verlangsamen. Durch diesen Effekt in Kombination mit der Multireflexion der Photonen erhöht sich die Kontaktwahrscheinlichkeit zwischen Photon und Luftpartikel noch weiter. Die mit den Pfeilen 8 angedeutete vorteilhafte Strömungssituation trägt ebenfalls zu der optimalen Funktionsweise bei.

Im Ergebnis wird erfindungsgemäß eine gegenüber dem Stand der Technik deutlich erhöhte Entkeimungseffizienz bei konstantem Energieeintrag erreicht. Um Umkehrschluss wird es möglich, mit vergleichsweise geringerer optischer Strahlung ein gleiches Entkeimungsergebnis zu erzielen (Energieeinsparung). Dadurch kann auch der Einsatz von LEDs als umweltschonendere Technologie im Vergleich zu quecksilberbelasteter Niederdruckstrahler gerechtfertigt werden.

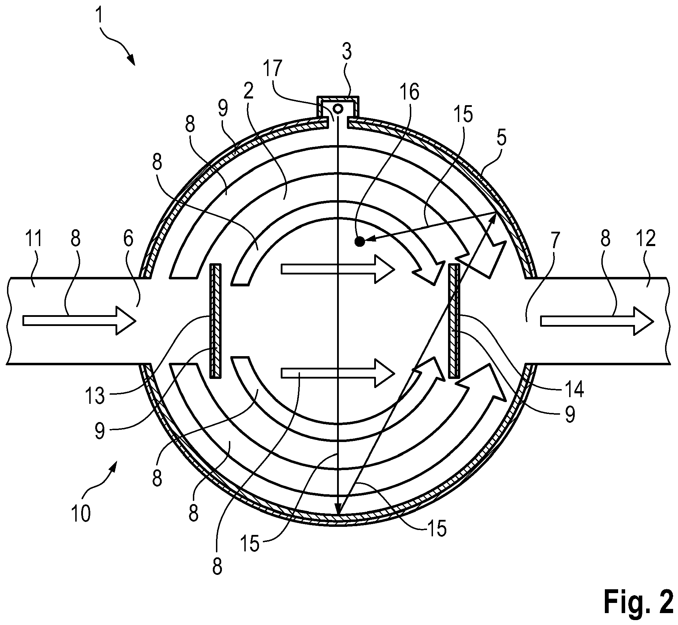

Alternativ oder zusätzlich dazu, dass bei einer erfindungsgemäßen Luftreinigungseinrichtung 1 die oder wenigstens eine Beleuchtungseinheit 3 innerhalb des Luftbehandlungsraumes 2 angeordnet ist, ist es natürlich auch möglich, dass sich die oder wenigstens eine Beleuchtungseinheit 3 außerhalb befindet und von außen in diesen einstrahlt. Ein Ausführungsbeispiel hierfür ist- wiederum rein schematisch - in der Figur 2 gezeigt. Dieses unterscheidet sich von demjenigen gemäß Figur 1 dadurch, dass die eine hier vorgesehene Beleuchtungseinheit 3 außerhalb des Luftbehandlungsraums 3, konkret von außen an der den Luftbehandlungsraums 3 begrenzenden Wandung 5 positioniert ist. Die Beleuchtungseinheit 3 ist hier direkt an der Wandung 5 von außen befestigt. Es ist eine Lichteinlassöffnung 17 in der den Luftbehandlungsraum 2 begrenzenden Wandung 5 vorgesehen, durch welche von der Beleuchtungseinheit 3 emittierte Strahlung in den Luftbehandlungsraum 2 eingestrahlt werden kann. Die Beleuchtungseinheit 3 umfasst, wie die aus Figur 1, UV-Licht emittierende LEDs. Da sie sich nicht mittig innerhalb des kugelförmigen Luftbehandlungsraumes 2 befindet, sondern seitlich von diesem, ist sie nicht für eine Abstrahlung in alle Richtungen (4 pi) ausgebildet, sondern für eine Abstrahlung in den Halbraum (2 pi).

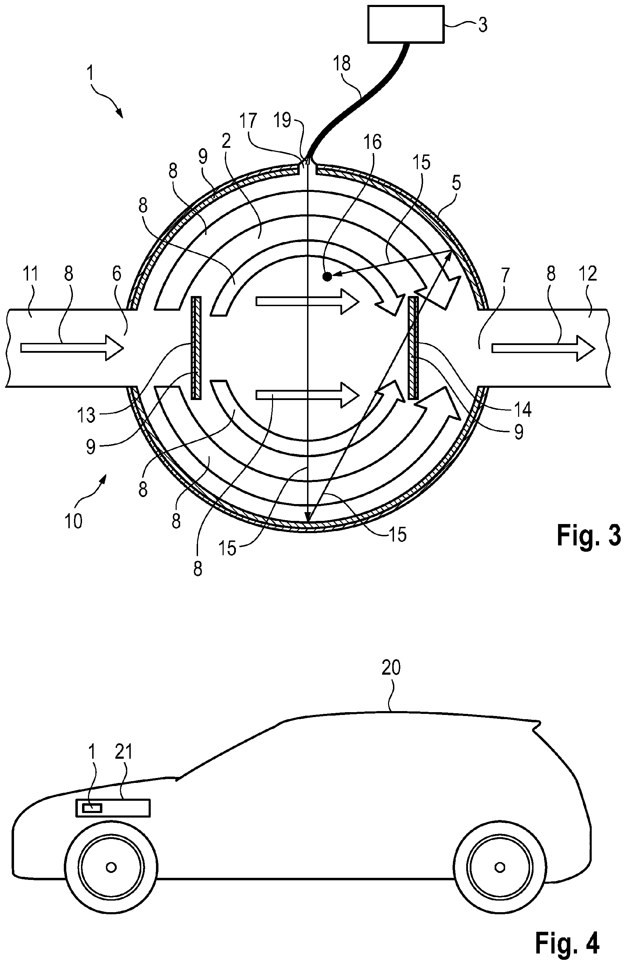

Ein weiteres Ausführungsbeispiel mit einer außerhalb des Luftreinigungsraumes angeordneten Beleuchtungseinheit 3 ist in der Figur 3 dargestellt. Im Unterschied zu dem Beispiel gemäß Figur 2 ist die Beleuchtungseinheit 3 hier über ein optisches Medium, konkret ein Glasfaserkabel 18 mit dem Luftbehandlungsraum 2 gekoppelt. Über das Glasfaserkabel 18, welches dazu ausgebildet ist, die von der Beleuchtungseinheit 3 emittierte Infrarotstrahlung zu führen bzw. zu leiten, kann diese zu dem Luftbehandlungsraum 2 gelangen. Dazu ist das eine freie Ende des Glasfaserkabels 18 mit der Beleuchtungseinheit 3 und das andere freie Ende mit einem außenseitig zu diesem Zweck an der Wandung 5 vorgesehen Glasfaserkable-Anschluss 19 verbunden. Die aus dem Glasfaserkabel 18 austretende Infrarotstrahlung tritt in den Luftbehandlungsraum 2 über eine in der Wandung 5 vorgesehene Öffnung 17 ein und kann dann, genau wie bei den vorangegangenen Beispielen, in diesem vielfach reflektiert werden.

Da die Beleuchtungseinheit 3 bei den Ausführungsbeispielen gemäß den Figuren 2 und 3 außerhalb des Luftbehandlungsraumes 2 positioniert ist, kann sie bei Bedarf sehr einfach gewartet bzw. ausgetauscht werden. Sie ist ferner dem Luftstrom durch den Luftbehandlungsraum 2 nicht ausgesetzt und blockiert den Strahlengang durch diesen nicht. Da sich bei diesem Beispiel keine stromführenden Komponenten in dem Luftbehandlungsraum 2 befinden, sind keine Stromzuleitungen in diesen erforderlich, was Schutzmaßnahmen gegen Feuchtigkeit und elektrischen Schlag reduziert. Im Gegenzug sei erwähnt, dass zweckmäßiger Weise Sicherheitskonzepte zur Verhinderung von Einsehen in die unsichtbare UV-Strahlung bei Installation und Wartung der Luftreinigungseinrichtung vorhanden sein sollten.

Die erfindungsgemäßen Luftreinigungseinrichtungen 1 eignen sich für die verschiedensten Anwendungsgebiete. Sie können überall dort genutzt werden, wo eine Entkeimung der Luft vorteilhaft oder erforderlich ist. Sie können zum Beispiel Bestandteil einer raumlufttechnischen Anlage, Lüftungs- oder Klimaanlage sein.

Sie eignen sich sowohl für den stationären Bereich, können beispielsweise in einem Gebäude vorgesehen sein, als auch für den mobilen Bereich. Dabei können sie als Teil einer bestehenden raumlufttechnischen Anlage bzw. Lüftungs- und Klimaanlage sein, sowie fix oder mobil als eigenständiges System fungieren. Sie können auch Bestandteil eines erfindungsgemäßen Fahrzeugs 20 sein. Ein Ausführungsbeispiel eines solchen ist - rein schematisch in Teilschnittdarstellung - in Figur 4 dargestellt. Es handelt sich um einen PKW 20 mit einer in diesen eingebauten Klimaanlage 21, die eine erfindungsgemäße Luftreinigungseinrichtung 1 umfasst, beispielsweise eine solche, wie sie in Figur 1, 2 oder Figur 3 dargestellt ist.

Alternativ dazu, dass es sich bei einem erfindungsgemäßen Fahrzeug um einen PKW handelt, kann ein solches beispielsweise auch durch einen LKW oder Bus oder durch ein Luft- oder Wasserfahrzeug, etwa Flugzeug oder Schiff, gegeben sein.

## Patentansprüche

1. Luftreinigungseinrichtung (1) für die Luftentkeimung mit einem Luftbehandlungsraum (2) und wenigstens einer Beleuchtungseinheit (3), die derart ausgebildet und angeordnet ist, dass mittels dieser UV-Strahlung in den Luftbehandlungsraum (2) gestrahlt werden kann, um zu reinigende Luft, die im Betrieb den Luftbehandlungsraum (2) durchströmt, mit dieser zu behandeln und so darin enthaltene Mikroorganismen, insbesondere Bakterien und/oder Viren und/oder Pilze, abzutöten oder zu inaktivieren, **dadurch gekennzeichnet, dass** der Luftbehandlungsraum (2) zumindest abschnittsweise zumindest im Wesentlichen kugelförmig ausgebildet ist und die den Luftbehandlungsraum (2) begrenzende Wandung (5) innenseitig zumindest abschnittsweise mit einer Reflexionsbeschichtung (9), die dazu ausgebildet ist, von der wenigstens einen Beleuchtungseinheit (3) emittierte UV-Strahlung bevorzugt diffus zu reflektieren, versehen ist.

2. Luftreinigungseinrichtung (1) nach Anspruch 1, **dadurch gekennzeichnet, dass** die oder wenigstens eine der Beleuchtungseinheiten (3) wenigstens eine LED (4) umfasst, die bevorzugt ausgebildet ist, elektromagnetische Strahlung im UV-Bereich, insbesondere elektromagnetische Strahlung mit einer Wellenlänge im Wellenlängenbereich von 200 nm bis 400 nm, bevorzugt 200 nm bis 380 nm, besonders bevorzugt 230 nm bis 285 nm, zu emittieren, und/oder dass die oder wenigstens eine der Beleuchtungseinheiten (3) wenigstens einen Excimer-Strahler umfasst, der bevorzugt ausgebildet ist, elektromagnetische Strahlung im UV-Bereich, insbesondere elektromagnetische Strahlung mit einer Wellenlänge im Wellenlängenbereich von 170 nm bis 400 nm, bevorzugt 170 nm bis 380 nm, besonders bevorzugt 210 nm bis 285 nm, zu emittieren, und/oder dass die oder wenigstens eine der Beleuchtungseinheiten (3) wenigstens eine Quecksilbemiederdrucklampe umfasst oder durch eine solche gegeben ist.

3. Luftreinigungseinrichtung (1) nach Anspruch 1 oder 2, **dadurch gekennzeichnet, dass** die Reflexionsbeschichtung (9) Polytetrafluorethylen umfasst oder aus Polytetrafluorethylen besteht, und/oder dass die Reflexionsbeschichtung (9) Bariumsulfat umfasst oder aus Bariumsulfat besteht und/oder dass die Reflexionsbeschichtung (9) mit Siliziumoxyd beschichtetes Aluminium umfasst oder daraus besteht.

4. Luftreinigungseinrichtung (1) nach einem der vorhergehenden Ansprüche, **dadurch gekennzeichnet, dass** die Reflexionsbeschichtung (9) dazu ausgebildet ist wenigstens 50 %, bevorzugt wenigstens 70 %, besonders bevorzugt wenigstens 90 %, ganz besonders bevorzugt wenigstens 95 % derjenigen Strahlung zu reflektieren, die von der oder wenigstens einer der Beleuchtungseinheiten emittiert wird.

5. Luftreinigungseinrichtung (1) nach einem der vorhergehenden Ansprüche, **dadurch gekennzeichnet, dass** der Luftbehandlungsraum (2) eine Lufteinlassöffnung (6) und eine der Lufteinlassöffnung (6) bevorzugt gegenüberliegende Luftauslassöffnung (7) aufweist, insbesondere, wobei innerhalb des Luftbehandlungsraumes (2) gegenüber der Lufteinlassöffnung (6) ein Einlass-Shutterelement (13) angeordnet ist, bevorzugt, wobei das Einlass-Shutterelement (13) an seiner von der Lufteinlassöffnung (6) abgewandten Seite mit einer Reflexionsbeschichtung (9), die dazu ausgebildet ist, von der wenigstens einen Beleuchtungseinheit (3) emittierte UV-Strahlung bevorzugt diffus zu reflektieren, versehen ist, und/oder dass innerhalb des Luftbehandlungsraumes (2) gegenüber der Luftauslassöffnung (7) ein Auslass-Shutterelement (14) angeordnet ist, bevorzugt, wobei das Auslass-Shutterelement (14) an seiner von der Luftauslassöffnung (7) abgewandten Seite mit einer Reflexionsbeschichtung (9), die dazu ausgebildet ist, von der wenigstens einen Beleuchtungseinheit emittierte UV-Strahlung bevorzugt diffus zu reflektieren, versehen ist, bevorzugt, wobei die oder wenigstens eine der Beleuchtungseinheiten (3) zwischen dem Einlass-Shutterlement (13) und dem Auslass-Shutterlement (14) angeordnet ist.

6. Luftreinigungseinrichtung (1) nach einem der vorhergehenden Ansprüche, **dadurch gekennzeichnet, dass** der Luftbehandlungsraum (2) einen bevorzugt zentralen Abschnitt eines Strömungskanals (10) der Einrichtung (1) bildet.

7. Luftreinigungseinrichtung (1) nach Anspruch 6, **dadurch gekennzeichnet, dass** der Strömungskanal (10) einen dem Luftbehandlungsraum (2) in Strömungsrichtung vorgeschalteten, bevorzugt zylinderförmigen Luftzuführabschnitt (11) und einen dem Luftbehandlungsraum (2) in Strömungsrichtung nachgeschalteten, bevorzugt zylinderförmigen Luftabführabschnitt (12) umfasst, bevorzugt, wobei der Durchmesser des Luftzuführabschnitts (11) kleiner ist als der Durchmesser des Luftbehandlungsraumes (2), und/oder dass der Durchmesser des Luftabführabschnitts (12) kleiner ist als der Durchmesser des Luftbehandlungsraumes (2).

8. Luftreinigungseinrichtung (1) nach den Ansprüchen 5 und 7, **dadurch gekennzeichnet, dass** der Luftzuführabschnitt (11) in die Lufteinlassöffnung (6) des Luftbehandlungsraums (2) mündet und der Luftabführabschnitt (12) von der Luftauslassöffnung (7) des Luftbehandlungsraums (2) abgeht.

9. Luftreinigungseinrichtung (1) nach einem der vorhergehenden Ansprüche, **dadurch gekennzeichnet, dass** die oder wenigstens eine der Beleuchtungseinheiten (3) innerhalb des Luftbehandlungsraumes (2) angeordnet ist.

10. Luftreinigungseinrichtung (1) nach einem der vorhergehenden Ansprüche, **dadurch gekennzeichnet, dass** die oder wenigstens eine der Beleuchtungseinheiten (3) außerhalb des Luftbehandlungsraumes (2) angeordnet ist, insbesondere, wobei wenigstens eine Lichteinlassöffnung (17) in der den Luftbehandlungsraum (2) begrenzenden Wandung (5) vorgesehen ist, durch welche von der oder wenigstens einer der Beleuchtungseinheiten (3) emittierte Strahlung in den Luftbehandlungsraum (2) eingestrahlt werden kann, und/oder dass wenigstens ein optisches Medium (18), insbesondere wenigstens ein Lichtleiter, bevorzugt Glasfaserkabel der Glasstab, vorgesehen ist, über das Strahlung, die von der oder wenigstens einer der außerhalb des Luftbehandlungsraumes (2) angeordneten Beleuchtungseinheiten (3) emittiert wird, in den Luftbehandlungsraum (2) geführt werden kann.

11. Luftreinigungseinrichtung (1) nach einem der vorhergehenden Ansprüche, **dadurch gekennzeichnet, dass** wenigstens eine der wenigstens einen Beleuchtungseinheit (3) zugeordnete Kühleinrichtung vorgesehen ist, bevorzugt, wobei die wenigstens eine Kühleinrichtung außerhalb des Luftbehandlungsraumes (2) angeordnet ist, und/oder dass wenigstens eine der wenigstens einen Beleuchtungseinheit (3) zugeordnete Anschlussleitung vorgesehen ist, bevorzugt, wobei die wenigstens eine Anschlussleitung außerhalb des Luftbehandlungsraumes (2) angeordnet ist, und/oder dass der Luftbehandlungsraum (2) durch eine zumindest abschnittsweise zumindest im Wesentlichen hohlkugelförmige Wandung (5) begrenzt.

12. Raumlufttechnische Anlage umfassend wenigstens eine Luftreinigungseinrichtung (1) nach einem der vorhergehenden Ansprüche.

13. Lüftungs- oder Klimaanlage (21) umfassend wenigstens eine Luftreinigungseinrichtung (1) nach einem der Ansprüche 1 bis 11.

14. Fahrzeug (20), insbesondere PKW oder LKW oder Bus oder Flugzeug oder Schiff, umfassend wenigstens eine Luftreinigungseinrichtung (1) nach einem der Ansprüche 1 bis 11.

15. Verwendung einer Ulbricht-Kugel in einer Luftreinigungseinrichtung (1) für die Luftentkeimung mittels UV-Strahlung.
